# FASCICULE DE BREVET EUROPEEN

(11) **EP 1 275 346 B1**
(45) Date de publication et mention de la délivrance du brevet: **30.08.2006**
(21) Numéro de dépôt: 02356130.1
(22) Date de dépôt: 05.07.2002
(51) Int. Cl.: A61B 17/17

(54) **Ancillaire de pose d'un composant huméral de prothèse de coude**
Hilfsvorrichtung zur Positionierung eines Oberarmteils einer Ellenbogenprothese
Aid for positioning a humeral component of an elbow prosthesis

(30) Priorité: 09.07.2001 FR 0109099
(43) Date de publication de la demande: 15.01.2003
(60) Demande divisionnaire: 06013694.2
(73) Titulaire: TORNIER, 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 0 132 284
- US-A- 4 242 758
- US-A- 4 718 414

## Description

L'invention a trait à un ancillaire de pose d'un composant huméral de prothèse totale ou partielle de coude.

Il est connu de US-A-4 718 414 d'utiliser un guide de découpe osseuse pour le remplacement d'une surface articulaire de coude.

Le préambule de la revendication 1 est basé sur l'outil de guidage d'un poinçon dans la trochlée qui est connu de US-A-4 242 758.

Une prothèse de coude, telle que connue de EP-A-1 051 954, comprend un composant huméral qui doit reproduire la trochlée anatomique au moyen d'une trochlée prothétique. Le positionnement de cette trochlée prothétique par rapport à l'articulation est essentiel pour un bon fonctionnement du coude appareillé.

Jusqu'à présent, les chirurgiens implantent le composant huméral d'une prothèse de coude en faisant l'hypothèse que la position de l'axe de flexion de l'articulation peut être déterminée empiriquement à partir de celle du canal médullaire. Or, ceci n'est pas rigoureusement exact et il en résulte des défauts de positionnement de la trochlée prothétique par rapport aux surfaces articulaires cubitale et radiale avec lesquelles elle doit coopérer, qu'il s'agisse de surfaces anatomiques ou de surfaces prothétiques.

C'est à ces inconvénients qu'entend plus particulièrement remédier l'invention en proposant un ancillaire de pose qui permet un positionnement précis de la trochlée prothétique et, ainsi, améliore les conditions de fonctionnement du coude appareillé.

Dans cet esprit, l'invention concerne un ancillaire du type précité qui comprend un outil de guidage permettant le perçage de la trochlée humérale selon l'axe géométrique de flexion de l'articulation à équiper, cet outil comprenant un étrier globalement en forme de U apte à être immobilisé sur la trochlée, alors que l'outil définit une zone de guidage pour l'introduction d'un foret, d'un poinçon ou analogue dans la trochlée, selon l'axe de flexion de l'articulation.

Grâce à l'invention, le chirurgien peut, avant de procéder à une resection de l'extrémité inférieure de l'humérus, déterminer précisément l'axe de flexion de l'articulation et, grâce au perçage réalisé au moyen de l'outil de guidage, il peut mettre en place une broche qui lui sert ensuite d'axe physique de référence pour les mesures ultérieures et la mise en place du composant huméral prothétique.

Selon des aspects avantageux mais non obligatoires de l'invention, cet ancillaire incorpore une ou plusieurs des caractéristiques suivantes :
- L'outil précité comprend une vis creuse apte à exercer sur la trochlée un effort d'immobilisation de l'outil, en coopération avec une partie de l'étrier. Dans ce cas, la partie précitée de l'étrier est avantageusement pourvue de picots d'immobilisation par rapport à la trochlée et d'une encoche d'engagement de l'épitrochlée.
- Il comprend un gabarit de positionnement de l'outil de guidage par rapport à la face externe de la trochlée. Ce gabarit permet de déterminer la zone d'appui d'une vis creuse sur le condyle, c'est-à-dire le point d'introduction du foret ou du poinçon dans l'épicondyle anatomique.
- Il comprend une broche apte à être introduite partiellement dans le canal médullaire huméral, sensiblement selon l'axe diaphysaire, et au moins un gabarit étagé de détermination du déport antérieur de l'axe de flexion par rapport à l'axe diaphysaire, ce gabarit étant apte à coulisser le long de la broche précitée. Ce gabarit peut être une pièce monobloc pourvue d'un perçage central d'un diamètre correspondant au diamètre de la partie de la broche précitée dépassant du canal médullaire, cette pièce étant équipée de trois surfaces annulaires centrées sur ce perçage et susceptibles de venir en appui contre une broche en place dans la trochlée, selon l'axe de flexion. On peut prévoir que le gabarit porte des mentions correspondant à des références pré-établies de valeurs du déport antérieur et qu'il est prévu en plusieurs tailles correspondant respectivement à des tailles prédéterminées de trochlées standard.
- Plusieurs trochlées fantômes peuvent être prévues, qui sont aptes à être utilisées pour déterminer la taille approximative de la trochlée anatomique de l'articulation à équiper. Ces trochlées fantômes permettent de déterminer quel gabarit étagé doit être utilisé pour la détermination du déport antérieur précité et/ou quel gabarit de positionnement doit être utilisé pour la mise en place de l'outil de guidage.
- Il comprend un outil de guidage pour la découpe de l'humérus, cet outil incluant un support apte à être positionné par rapport à une broche en place dans la trochlée suivant l'axe de flexion précité et un gabarit apte à être monté sur le support en définissant au moins une zone de guidage d'un outil de découpe.

L'invention permet la pose d'un composant huméral de prothèse de coude selon une méthode qui comprend des étapes consistant à déterminer la position géométrique de l'axe de flexion de l'articulation à équiper par rapport à la trochlée humérale, à percer cette trochlée selon cet axe et à insérer une première broche dans cette trochlée selon cet axe. Grâce à cette méthode, la trochlée prothétique peut être positionnée de façon précise et interagir dans les meilleures conditions avec les surfaces articulaires complémentaires.

Selon le cas, cette méthode peut comprendre des étapes supplémentaires consistant à introduire partiellement une seconde broche dans le canal médullaire huméral, sensiblement selon l'axe diaphysaire, et à mesurer le déport antérieur de l'axe de flexion par rapport à l'axe diaphysaire, en faisant coulisser un gabarit étagé le long de la seconde broche jusqu'à ce qu'il vienne en contact avec la première.

Selon un autre aspect de cette méthode, on peut sélectionner, dans un jeu de trochlées fantômes, la trochlée dont la géométrie est la plus proche de la trochlée anatomique et sélectionner le gabarit étagé en fonction de la trochlée fantôme déjà sélectionnée.

Par ailleurs, il est possible de prévoir des étapes supplémentaires consistant à positionner, sur la première broche en place dans la trochlée selon l'axe de flexion, un outil de guidage pour la découpe de l'humérus et à découper l'humérus selon un profil déterminé par cet outil de guidage.

Enfin, on peut prévoir de déterminer l'angle entre l'axe de flexion et la projection sur cet axe de l'axe diaphysaire huméral.

L'invention sera mieux comprise et d'autres avantages de celle-ci apparaîtront plus clairement à la lumière de la description qui va suivre d'un mode de réalisation d'un ancillaire de pose conforme à son principe et de sa mise en oeuvre, donnée uniquement à titre d'exemple et faite en référence aux dessins annexés dans lesquels :
- la figure 1 est une vue en perspective de l'extrémité inférieure d'un humérus, lors d'une première étape de pose d'un composant huméral grâce à un ancillaire conforme à l'invention, des éléments annexes étant représentés à plus petite échelle ;
- la figure 2 est une vue analogue à la figure 1, avec arrachements partiels, lors d'une seconde étape ;
- la figure 3 est une vue analogue à la figure 1 lors d'une troisième étape ;
- la figure 4 est une vue analogue à la figure 1 lors d'une quatrième étape ;
- la figure 5 est une coupe selon la ligne V-V à la figure 4 ;
- la figure 6 est une vue en perspective lors d'une cinquième étape d'utilisation de l'ancillaire ;
- le figure 7 est une vue en perspective lors d'une sixième étape d'utilisation de l'ancillaire et
- la figure 8 est une vue en perspective lors de la mise en place d'une trochlée prothétique d'essai.

L'extrémité inférieure de l'humérus H représentée sur les figures comprend une trochlée anatomique T constituant la surface articulaire inférieure S de l'humérus. Cette surface S est partiellement cylindrique et centrée sur un axe imaginaire X-X' qui constitue l'axe géométrique de flexion de l'articulation du coude.

Dans une première étape d'utilisation de l'ancillaire de l'invention, on compare la trochlée anatomique T avec trois trochlées fantômes 10₁, 10₂, 10₃ de tailles différentes. Par exemple, la trochlée 10₁ est de taille inférieure à la trochlée 10₂ qui est elle-même de taille inférieure à la trochlée 10₃. Du fait de cette comparaison, on sélectionne l'une des trois trochlées fantômes, par exemple la trochlée 10₂, comme étant celle qui est la plus proche sur le plan géométrique de la trochlée anatomique T.

En fonction de la trochlée fantôme sélectionnée, dans l'exemple de la trochlée 10₂, on sélectionne un gabarit de positionnement 20₂ dans un jeu de trois gabarits 20₁, 20₂, 20₃ correspondant respectivement aux trochlées 10₁ à 10₃. Le gabarit 20 peut également être sélectionné en apposant son extrémité 21 sur le condyle huméral et en contrôlant son adéquation avec le profil de ce condyle.

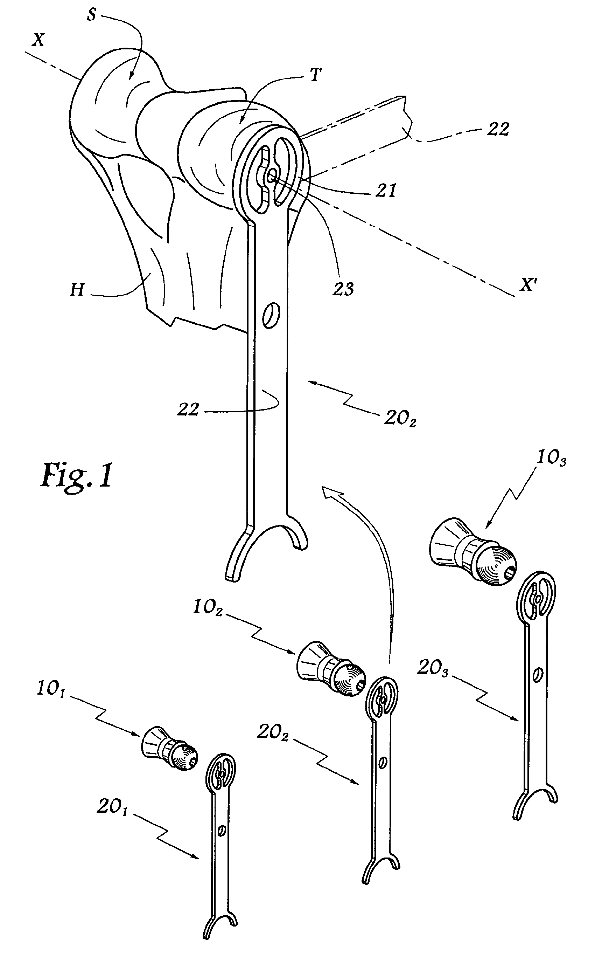

Pour la clarté du dessin, les éléments 10₁ à 10₃ et 20₁ à 20₃ sont représentés, en partie inférieure de la figure 1, à plus petite échelle que l'humérus H et le gabarit 20₂ en partie supérieure.

Le gabarit 20₂ sélectionné est alors apposé sur le côté de l'humérus H de telle sorte que son extrémité 21 de forme arrondie est en appui sur le condyle externe de l'humérus. En pratique, le manche 22 du gabarit 20₂ peut être disposé parallèlement à l'humérus H, comme représenté en traits pleins à la figure 1, ou perpendiculairement à celui-ci, comme représenté en traits mixtes.

L'extrémité 21 du gabarit 20₂ est positionnée de telle sorte qu'elle permet de repérer, avec une précision acceptable, le centre de la trochlée T par lequel passe l'axe X-X'. Cette extrémité 21 est pourvue d'un perçage central 23 dans lequel peut être insérée une pointe de marquage du condyle considéré. Le gabarit 20₂ permet donc de repérer la trace de l'axe X-X' sur le condyle de la trochlée T.

Il est alors possible d'utiliser un outil 30 de guidage pour le perçage de la trochlée T selon l'axe X-X'. Cet outil 30 comprend un étrier 31 globalement en forme de U et qui comprend une première branche 32 et une seconde branche 33 reliées par un fond 34. L'extrémité 32a de la branche 32 est globalement parallèle au fond 34 et est équipée d'un taraudage 32b prolongé par un lamage de centrage 32 permettant le guidage et la réception d'une vis 35 dont la tête 35a est pourvue d'un orifice polygonal 35b permettant son entraînement par une clef mâle adaptée.

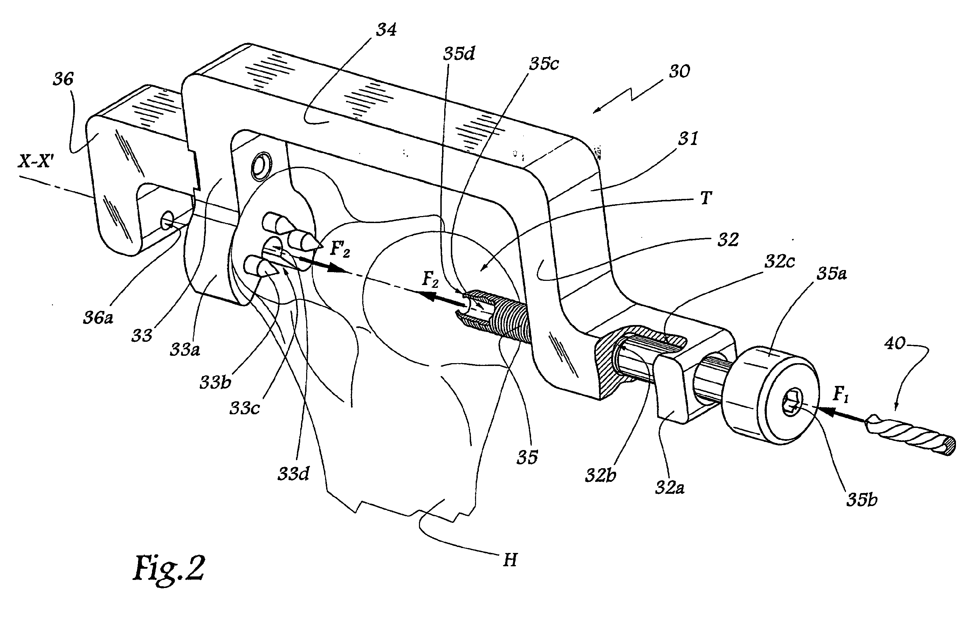

La vis 35 est creuse en ce sens qu'elle est pourvue d'un canal longitudinal 35c qui s'étend sur toute sa longueur et qui est visible par arrachement à la figure 2.

La seconde branche 33 de l'étrier 31 se termine par une extrémité 33a pourvue de trois picots 33b et d'une encoche 33c destinée à recevoir l'épitrochlée de l'humérus H qui est représenté en fantôme à la figure 2 afin de faciliter la visualisation de l'outil 30.

Une branche accessoire 36 est rapportée sur l'étrier 31 et s'étend à partir de la branche 33 selon une direction globalement parallèle au fond 34, puis selon une direction globalement perpendiculaire à ce fond. La branche 36 est percée d'un orifice 36a sensiblement aligné avec un orifice 33d prévu dans l'extrémité 33a de la branche 33 et aligné précisément avec le canal 35c de la vis 35 lorsque celle-ci est en place dans le taraudage 32b. L'orifice 36a permet une visée de l'axe de flexion par la face interne de l'humérus, lorsque cela est nécessaire.

L'outil 30 est mis en place sur l'humérus H en pointant l'extrémité avant 35d de la vis 35 sur la marque réalisée grâce au gabarit 20₁, 20₂ ou 20₃ utilisé et en disposant l'encoche 33c autour de l'épitrochlée de l'humérus H...Dans cette position, l'axe commun aux orifices 36a, 33d et au canal 35c est globalement aligné avec l'axe de flexion X-X', tel qu'il est défini par la trochlée T.

Il est alors possible de percer la trochlée T grâce à un foret 40 traversant la vis 35, l'extrémité 33a et l'orifice 36a. La flèche F₁ représente la direction d'introduction du foret 40 dans le canal 35c, l'orifice 33d et l'orifice 36a.

A la place du foret 40, on peut utiliser un poinçon ou tout autre outil adapté pour percer la trochlée T selon l'axe X-X'. Le forêt 40 peut également être introduit par l'orifice 36a, au choix du chirurgien opérateur.

On note qu'une immobilisation ferme de l'outil 30 par rapport à l'humérus peut être obtenue par la coopération de la vis 35 et des picots 33 qui exercent un effort F₂, F'₂ de compression sur l'humérus H, selon une direction globalement parallèle à l'axe X-X'.

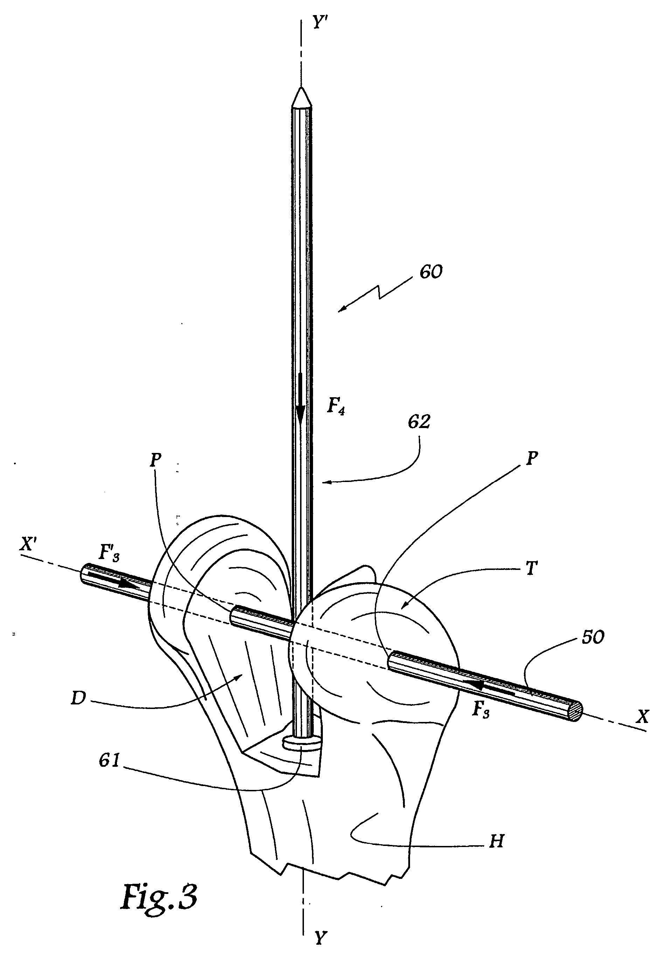

Lorsque le perçage précité a été réalisé, il est possible de retirer l'outil 30 en desserrant la vis 35, puis de réaliser dans l'humérus H une découpe D d'accès au canal médullaire de l'humérus, cette découpe étant visible à la figure 3.

Il est alors possible d'insérer dans le perçage P réalisé grâce au foret 40 une broche 50 qui devient alors la matérialisation physique de l'axe géométrique X-X'. Les flèches F₃ et F'₃ représentent les directions d'introduction de la broche 50 dans le perçage P, selon l'axe X-X'. On peut également insérer dans le canal médullaire de l'humérus H une broche étagée 60 comprenant une première section 61 de diamètre relativement important, par exemple égal à 6 mm, et une seconde section 62 de diamètre plus faible, par exemple de l'ordre de 3 mm. On peut faire l'hypothèse que la broche 60 s'étend sensiblement selon l'axe diaphysaire Y-Y' de l'humérus. La flèche F₄ représente la direction d'introduction de la broche 60 dans le canal médullaire.

Il convient alors de déterminer au moins approximativement le déport antérieur d entre les axes X-X' et Y-Y'. Ce déport, parfois appelé « offset antérieur », correspond à la distance entre les axes X-X' et Y-Y' selon une direction perpendiculaire à ces deux axes.

Il est essentiel de connaître la valeur de ce déport pour sélectionner une trochlée prothétique dont l'axe central pourra être correctement positionné par rapport à l'axe diaphysaire de l'humérus. Il est en effet possible de prévoir différentes trochlées prothétiques, avec des axes positionnés différemment, comme cela ressort, par exemple, de EP-A-1 051 954.

Pour procéder à la détermination du déport d, on utilise des gabarits 70₁, 70₂, 70₃ de forme étagée. Plus précisément, on sélectionne un gabarit en fonction de la trochlée fantôme préalablement sélectionnée, la trochlée 10₂ dans l'exemple décrit. Dans ce cas, on sélectionne le gabarit 10₂ de taille intermédiaire et on le fait coulisser autour de la broche 60, comme représenté par la flèche F₅. Les gabarits 70₁, 70₂ et 70₃ sont chacun pourvus d'un perçage central 71 de diamètre sensiblement égal à celui de la section 62 de la branche 60, ce qui leur permet de coulisser sans difficulté sur cette broche.

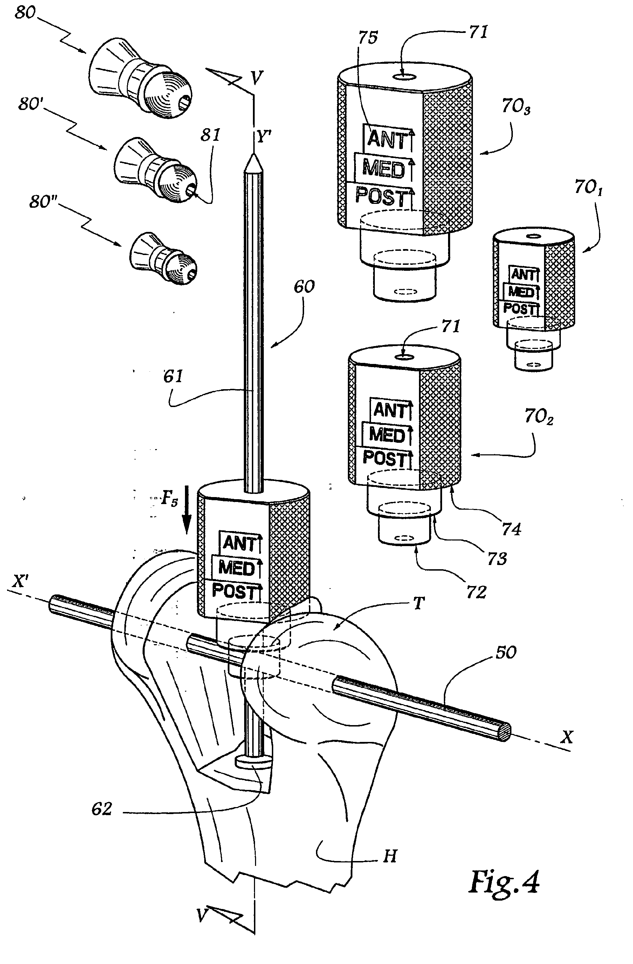

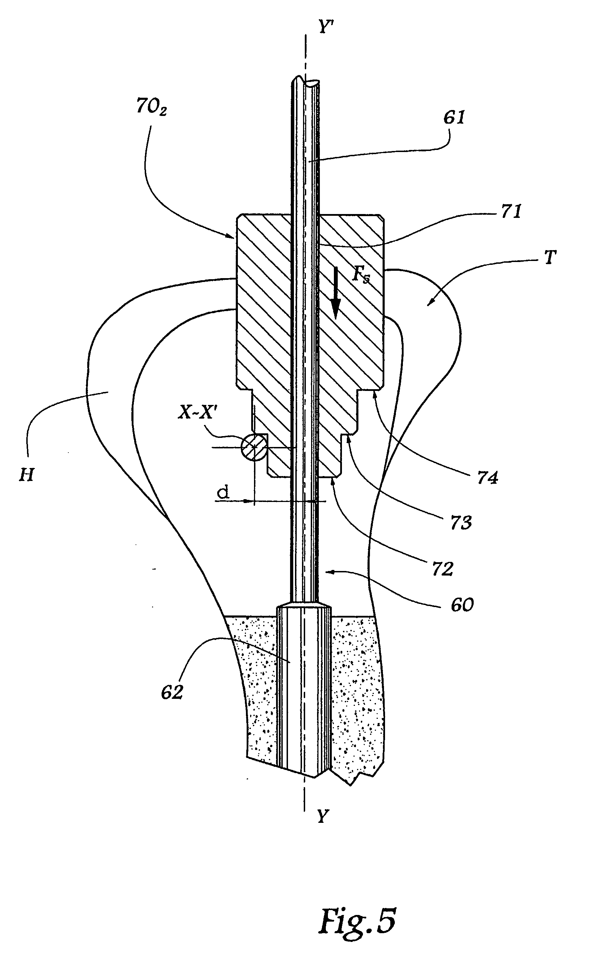

Le gabarit 70₂ est pourvu de trois surfaces annulaires 72, 73 et 74 dont le diamètre va croissant et qui sont chacune susceptibles de venir en appui contre la broche 50 dans la configuration des figures 4 et 5.

Les gabarits 70₁ et 70₂ présentent sensiblement la même géométrie, avec des surfaces annulaires étagées aptes à venir en appui contre la broche 50.

Chaque gabarit 70₁, 70₂, 70₃ porte des inscriptions 75 permettant de reconnaître immédiatement la valeur approximative du déport d de la façon suivante :
- ANT signifie « antérieur », c'est-à-dire que le déport antérieur est important
- MED signifie « médian », c'est-à-dire que le déport antérieur est moyen
- POST signifie « postérieur », c'est-à-dire que le déport antérieur est faible.

Ainsi, en fonction de la surface 72, 73 ou 74 en appui contre la broche 50, le chirurgien sait si le déport d doit être considéré comme important, moyen ou faible pour la suite de l'opération.

Le fait de repérer quelle surface 72, 73 ou 74 du gabarit 70₂ vient au contact de la broche 50 permet d'avoir une bonne idée de la valeur du déport d et de sélectionner en conséquence la trochlée prothétique devant équiper l'humérus H. Les différentes trochlées prothétiques 80, 80' et 80'' représentées à la figure 4 sont en effet caractérisées par le positionnement de leur perçage central 81 par rapport à leurs surfaces externes, ce perçage central permettant leur montage sur un élément ancré dans l'humérus, conformément à l'enseignement technique de EP-A-1 051 954.

Ainsi, l'utilisation du gabarit 70₂ permet au chirurgien de savoir laquelle des trochlées prothétiques 80, 80' ou 80'' il doit utiliser pour équiper l'humérus H

On comprend que trois ou plus de trois trochlées prothétiques peuvent être prévues en correspondance avec chaque taille de gabarit 70₁, 70₂, 70₃.

Bien entendu, le nombre de gabarits n'est pas limité à trois et dépend en pratique du nombre de prothèses fantômes 10₁, 10₂, 10₃ prévu.

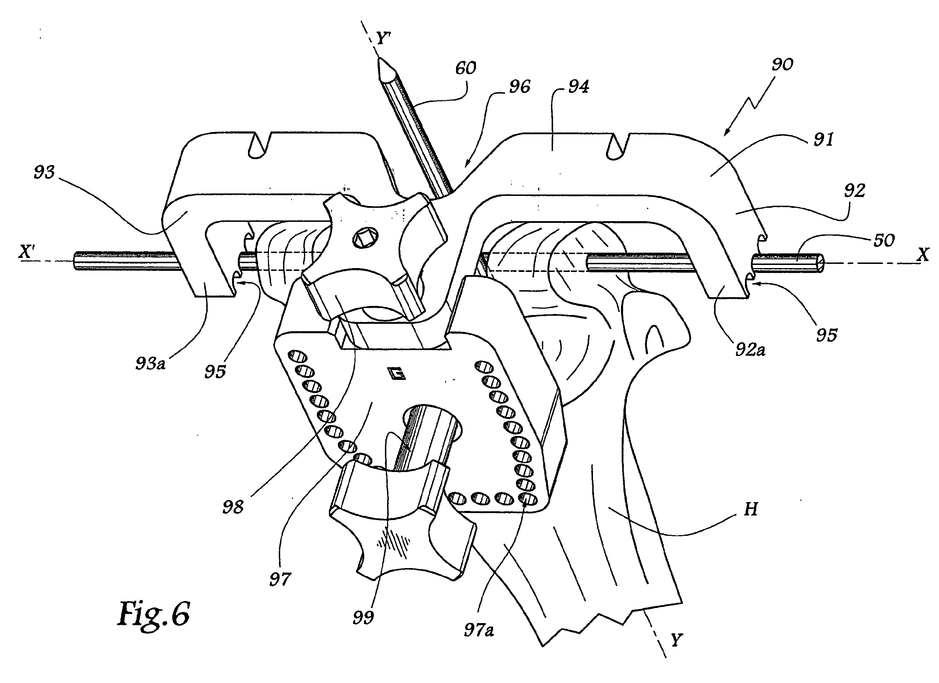

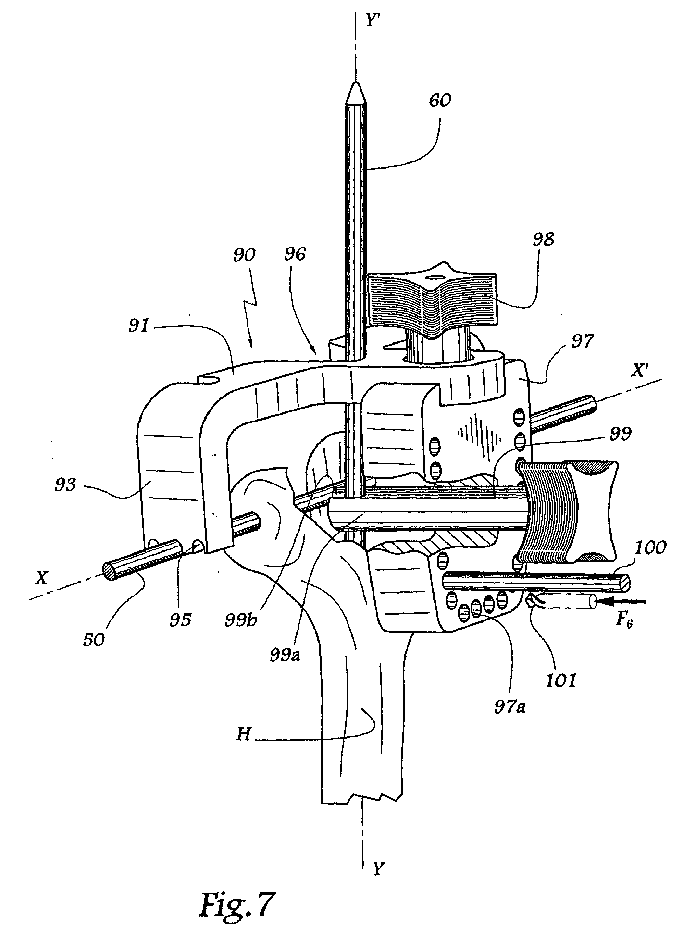

Lorsque le déport d a été déterminé, on utilise un outil de guidage 90 qui comprend un étrier 91 apte à venir en appui sur la broche 50, comme représenté aux figures 6 et 7. Cet étrier comporte deux branches 92 et 93 dont les extrémités respectives 92a et 93a sont pourvues d'encoches 95 de réception partielle de la broche 50, ce qui permet une bonne immobilisation de l'étrier 91 sur la broche 50.

Le fond 94 de l'étrier 91 est pourvu d'une découpe 96 de passage de la broche 60.

Un gabarit 97 est prévu pour être immobilisé sur l'étrier 91 grâce à une vis de blocage 98. Le gabarit 97 porte un axe coulissant 99 muni, à son extrémité libre 99a, d'une encoche 99b, telle que l'extrémité 99a peut être disposée autour de la broche 60. Ceci permet de déterminer un positionnement médio-latéral satisfaisant du gabarit 97.

Le gabarit 97 peut être monté sur l'étrier 91 dans deux positions correspondant respectivement à son utilisation pour une articulation de coude gauche ou de coude droit. Ainsi, un même outil 90 peut être utilisé pour les deux types de coudes susceptibles d'être équipés.

Le gabarit 97 est pourvu de plusieurs perçages 97a de passage de broches ou de forets permettant d'agrandir la découpe D aux dimensions de l'élément devant être ancré dans l'humérus H.

Comme représenté à la figure 7, on peut disposer dans certains perçages 97a une ou des broches 100 contribuant à une bonne immobilisation du gabarit 97 par rapport à l'humérus H, alors que l'on perce l'humérus H en insérant un foret 101 successivement dans les différents autres perçages 97a, comme représenté par la flèche F₆. Un poinçon ou tout autre outil adapté peut être utilisé à la place du foret 101.

Au terme de cette opération, on obtient dans l'humérus H une découpe D adaptée à la mise en place d'un élément prothétique huméral 110.

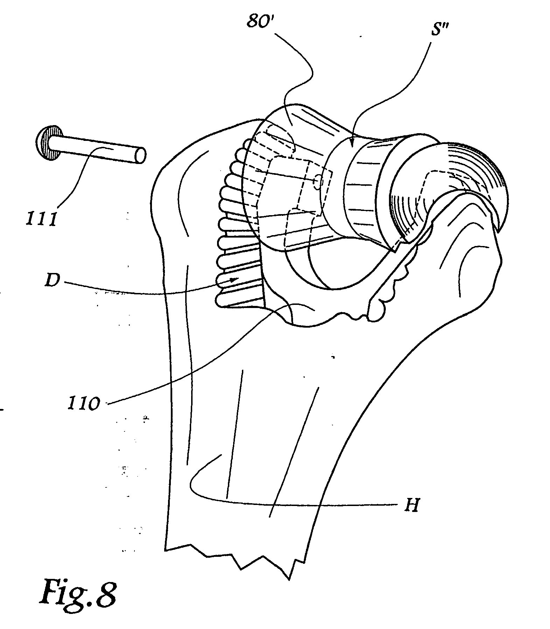

Après l'élargissement de la découpe D et avant la mise en place de l'élément 110, les broches 50 et 60 sont retirées et le perçage préalablement utilisé pour la broche 60 est agrandi pour la mise en place de l'élément 110, comme représenté à la figure 8. Cet élément peut recevoir une vis 111 d'immobilisation de la trochlée prothétique 80, 80' ou 80'' sélectionnée grâce au gabarit 70₂ utilisé précédemment.

Ainsi, la surface articulaire externe S'' de la trochlée 80' utilisée est positionnée de façon précise par rapport à la surface anatomique S. En particulier, la position de l'axe géométrique de la surface S'' est sensiblement confondue avec l'axe géométrique de flexion X-X'.

Lorsque l'outil 90 est en place sur la broche 50, on peut monter sur l'étrier 91 un rapporteur d'angle, non représenté, afin de mesurer l'angle entre l'axe X-X' et sa projection sur l'axe diaphysaire Y-Y'. La valeur de cet angle peut être utilisée pour déterminer la géométrie de l'élément 110.

En pratique, les trochlées prothétiques 80, 80' et 80'' sont des composants d'essai, le composant définitif étant mis en place à une étape ultérieure de la pose de la prothèse, en particulier après l'appareillage éventuel du cubitus et/ou du radius.

## Revendications

1. Ancillaire de pose d'un composant huméral de prothèse totale ou partielle de coude, comprenant un outil de guidage (30) permettant le perçage de la trochlée humérale (T) selon l'axe géométrique (X-X') de flexion de l'articulation à équiper, alors que ledit outil définit une zone (35c, 33d, 36a) de guidage pour l'introduction (F₁) d'un foret (40), d'un poinçon ou analogue, dans la trochlée selon ledit axe, **caractérisé en ce que** ledit outil comprend un étrier (31) globalement en forme de U, apte à être immobilisé (F₂, F'₂) sur la trochlée.

2. Ancillaire selon la revendication 1, **caractérisé en ce que** ledit outil (30) comprend une vis creuse (35) apte à exercer sur la trochlée (T) un effort (F₂, F'₂) d'immobilisation dudit outil, en coopération avec une partie (33a) dudit étrier (31).

3. Ancillaire selon la revendication 2, **caractérisé en ce que** ladite partie (33a) dudit étrier (31) est pourvue de picots (33b) d'immobilisation par rapport à la trochlée (T) et d'une encoche (33c) d'engagement de l'épitrochlée.

4. Ancillaire selon l'une des revendications précédentes, **caractérisé en ce qu'**il comprend un gabarit (20₁, 20₂, 20₃) de positionnement dudit outil de guidage (30) par rapport à la face externe de la trochlée (T).

5. Ancillaire selon l'une des revendications précédentes, **caractérisé en ce qu'**il comprend une broche (60) apte à être introduite partiellement dans le canal médullaire huméral, sensiblement selon l'axe diaphysaire (Y-Y'), et au moins un gabarit étagé (70₁, 70₂, 70₃) de détermination du déport antérieur (d) dudit axe de flexion (X-X') par rapport audit axe diaphysaire, ledit gabarit étant apte à coulisser le long de ladite broche.

6. Ancillaire selon la revendication 5, **caractérisé en ce que** ledit gabarit est une pièce monobloc (70₁, 70₂, 70₃) pourvue d'un perçage central (71) de diamètre correspondant au diamètre de la partie (62) de ladite broche dépassant dudit canal médullaire, ladite pièce étant équipée de trois surfaces annulaires (72, 73, 74) centrées sur ledit perçage et susceptibles de venir en appui contre une broche (50) en place dans la trochlée (T), selon ledit axe de flexion (X-X').

7. Ancillaire selon l'une des revendications 5 ou 6, **caractérisé en ce que** ledit gabarit (70₁, 70₂, 70₃) porte des mentions (75) correspondant à des références pré-établies de valeurs dudit déport antérieur (d).

8. Ancillaire selon l'une des revendications 5 à 7, **caractérisé en ce que** ledit gabarit (70₁, 70₂, 70₃) est prévu en plusieurs tailles correspondant à des tailles prédéterminées de trochlées standard (10₁, 10₂, 10₃).

9. Ancillaire selon l'une des revendications 4 ou 8, **caractérisé en ce qu'**il comprend plusieurs trochlées fantômes (10₁, 10₂, 10₃) aptes à être utilisées pour déterminer la taille approximative de la trochlée anatomique (T) de l'articulation à équiper, lesdites trochlées fantômes permettant de déterminer quel gabarit étagé (70₁, 70₂, 70₃) doit être utilisé pour la détermination dudit déport antérieur (d) et/ou quel gabarit de positionnement (20₁, 20₂, 20₃) doit être utilisé pour la mise en place dudit outil de guidage (30).

10. Ancillaire selon l'une des revendications précédentes, **caractérisé en ce qu'**il comprend un outil de guidage (90) pour la découpe (D) de l'humérus incluant un support (91) apte à être positionné par rapport à une broche (50) en place dans la trochlée (T) selon ledit axe de flexion (X-X') et un gabarit (97) apte à être monté sur ledit support et définissant au moins une zone (97a) de guidage d'un outil de découpe (101).

## Claims

1. Aid for positioning a humeral component of a complete or partial elbow prosthesis, comprising a guide tool (30) allowing the humeral trochlea (T) to be drilled following the geometric bending axis (X-X') of the joint to be fitted, where this tool defines a guide zone (35c, 33d, 36a) for introducing (F₁) a drill (40), an awl or similar into the trochlea following this axis, **characterised in that** this tool comprises a stirrup (31) in the overall form of a U able to be immobilised (F₂, F'₂) on the trochlea.

2. Aid according to claim 1, **characterised in that** this tool (30) comprises a hollow bolt (35) able to exert a force (F₂, F'₂) for immobilisation of this tool on the trochlea (T), working with a part (33a) of this stirrup (31).

3. Aid according to claim 2, **characterised in that** this part (33a) of the stirrup (31) is provided with spikes (33b) for immobilisation in relation to the trochlea (T) and a notch (33c) for engagement of the epitrochlea.

4. Aid according to one of the previous claims, **characterised in that** it comprises a gauge (20₁, 20₂, 20₃) for positioning this guide tool (30) in relation to the external face of the trochlea (T).

5. Aid according to one of the previous claims, **characterised in that** it comprises a pin (60) able to be introduced partially into the humeral medullar canal, more or less following the shaft axis (Y-Y'), and at least one staged gauge (70₁, 70₂, 70₃) for determination of the previous mismatch (d) of this bending axis (X-X') in relation to this shaft axis, this gauge being able to slide along this pin.

6. Aid according to claim 5, **characterised in that** this gauge is a solid block (70₁, 70₂, 70₃) provided with a central hole (71) with a diameter corresponding to the diameter of the part (62) of this pin exceeding this medullar canal, this part being fitted with three annular surfaces (72, 73, 74) centred on this hole able to be supported against a pin (50) in place in the trochlea (T) following this bending axis (X-X').

7. Aid according to one of claims 5 or 6, **characterised in that** this gauge (70₁, 70₂, 70₃) has indications (75) corresponding to preestablished references for the values of this previous mismatch (d).

8. Aid according to one of claims 5 to 7, **characterised in that** this gauge (70₁, 70₂, 70₃) is provided in several sizes corresponding to predetermined standard sizes of trochleas (10₁, 10₂, 10₃).

9. Aid according to one of claims 4 or 8, **characterised in that** it comprises several phantom trochleas (10₁, 10₂, 10₃) able to be used to determine the approximate size of the anatomical trochlea (T) of the joint to be fitted, these phantom trochleas allowing it to be determined which staged gauge (70₁, 70₂, 70₃) must be used to determine the previous mismatch (d) and/or which positioning gauge (20₁, 20₂, 20₃) must be used to put in place the guide tool (30).

10. Aid according to one of the previous claims, **characterised in that** it comprises a guide tool (30) for cutting (D) the humerus including a support (91) able to be positioned in relation to a pin (50) in place in the trochlea (T) following this bending axis (X-X') and a gauge (97) able to be fitted on this support and defining at least one guide zone (97a) of a cutting tool (101).

## Patentansprüche

1. Hilfsvorrichtung zur Positionierung eines Oberarmteils einer Gesamt- oder Teilprothese eines Ellenbogens, die eine Führungseinrichtung (30) umfasst, welche das Durchbohren der Oberarmtrochlea (T) entlang der geometrischen Beugeachse (X-X') des auszustattenden Gelenks ermöglicht, so dass die Einrichtung eine Führungszone (35c, 33d, 36a) für die Einführung (F₁) eines Bohrers (40), eines Vorstechers oder dergleichen in die Trochlea entlang der Achse definiert, **dadurch gekennzeichnet, dass** die Einrichtung einen im Allgemeinen U-förmigen Bügel (31) umfasst, der an der Trochlea befestigt (F₂, F₂') werden kann.

2. Hilfsvorrichtung nach Anspruch 1, **dadurch gekennzeichnet, dass** das Gerät (30) eine Hohlschraube (35) umfasst, die im Zusammenwirken mit einem Teil (33a) des Bügels (31) auf die Trochlea (T) eine Kraft (F₂, F'₂) zum Befestigen der Einrichtung ausüben kann.

3. Hilfsvorrichtung nach Anspruch 2, **dadurch gekennzeichnet, dass** der Teil (33a) des Bügels (31) mit Befestigungsstacheln (33b) gegenüber der Trochlea (T) und mit einer Kerbe (33c) zum Einrasten mit der Epitrochlea versehen ist.

4. Hilfsvorrichtung nach einem der vorhergehenden Ansprüche, **dadurch gekennzeichnet, dass** sie eine Positionierungsschablone (20₁, 20₂, 20₃) der Führungseinrichtung (30) bezüglich der Außenfläche der Trochlea (T) umfasst.

5. Hilfsvorrichtung nach einem der vorhergehenden Ansprüche, **dadurch gekennzeichnet, dass** sie einen Zapfen (60), der im Wesentlichen entlang der Diaphysärachse (Y-Y') teilweise in den Medulärkanal des Oberarms eingeführt werden kann, und mindestens eine abgestufte Schablone (70₁, 70₂, 70₃) zur Bestimmung des vorderen Versatzes (d) der Beugeachse (X-X') gegenüber der Diaphysärachse umfasst, wobei die Schablone entlang des Zapfens gleiten kann.

6. Hilfsvorrichtung nach Anspruch 5, **dadurch gekennzeichnet, dass** die Schablone aus einem Stück (70₁, 70₂, 70₃) mit einer Mittelbohrung (71) besteht, wobei der Durchmesser der Mittelbohrung dem Durchmesser des Teils (62) des durch den Medulärkanal vorstoßenden Zapfens entspricht und der Teil mit drei ringförmigen Flächen (72, 73, 74) versehen ist, die auf die Bohrung zentriert sind und an einem Zapfen (50) an der Stelle der Trochlea (T) entlang der Beugeachse (X-X') anliegen können.

7. Hilfsvorrichtung nach einem der Ansprüche 5 oder 6, **dadurch gekennzeichnet, dass** die Schablone (70₁, 70₂, 70₃) Angaben (75) trägt, die den vorher festgelegten Bezugswerten des vorderen Versatzes (d) entsprechen.

8. Hilfsvorrichtung nach einem der Ansprüche 5 bis 7, **dadurch gekennzeichnet, dass** die Schablone (70₁, 70₂, 70₃) in mehreren Größen entsprechend der vorbestimmten Größe der Standardtrochlea (10₁, 10₂, 10₃) vorgesehen ist.

9. Hilfsvorrichtung nach einem der Ansprüche 4 oder 8, **dadurch gekennzeichnet, dass** sie mehrere Fantomtrochleen (10₁, 10₂, 10₃) umfasst, die zum Bestimmen der ungefähren Größe der anatomischen Trochlea (T) des auszustattenden Gelenks verwendet werden können, wobei die Fantomtrochleen die Bestimmung ermöglichen, welche abgestufte Schablone (70₁, 70₂, 70₃) zur Bestimmung des vorderen Versatzes (d) eingesetzt werden muss und/oder welche Positionierungsschablone (20₁, 20₂, 20₃) für die Einbringung der Führungseinrichtung (30) eingesetzt werden muss.

10. Hilfsvorrichtung nach einem der vorhergehenden Ansprüche, **dadurch gekennzeichnet, dass** sie eine Führungseinrichtung (90) für das Abteilen des Oberarms (D), welche einen Träger (91) aufweist, der bezüglich eines Zapfens (50) an der Stelle der Trochlea (T) entlang der Beugeachse (X-X') in Position gebracht werden kann, und eine Schablone (97), die auf den Träger montiert werden kann und mindestens eine Führungszone (97a) einer Abteilvorrichtung (101) definiert, umfasst.
